# EUROPÄISCHE PATENTSCHRIFT

(11) **EP 1 268 385 B1**
(45) Veröffentlichungstag und Bekanntmachung des Hinweises auf die Patenterteilung: **27.02.2008**
(21) Anmeldenummer: 01913822.1
(22) Anmeldetag: 21.02.2001
(51) Int. Cl.: C07C 51/235

(54) **VERFAHREN ZUR HERSTELLUNG ALIPHATISCHER CARBONSÄUREN AUS ALDEHYDEN**
METHOD FOR PRODUCING ALIPHATIC CARBOXYLIC ACIDS FROM ALDEHYDES
PROCEDE DE PREPARATION D'ACIDES CARBOXYLIQUES ALIPHATIQUES A PARTIR D'ALDEHYDES

(30) Priorität: 04.03.2000 DE 10010769
(43) Veröffentlichungstag der Anmeldung: 02.01.2003
(73) Patentinhaber: OXEA Deutschland GmbH, 46147 Oberhausen (DE)
(72) Erfinder: SPRINGER, Helmut, 46539 Dinslaken (DE); HEYMANNS, Peter, 45147 Essen (DE)
(86) Internationale Anmeldenummer: PCT/EP2001/001946
(87) Internationale Veröffentlichungsnummer: WO 2001/066505

(56) Entgegenhaltungen:
- DE-B- 1 154 454

## Beschreibung

Die vorliegende Erfindung betrifft ein neues, nicht katalytisches Verfahren zur Herstellung aliphatischer Carbonsäuren aus Aldehyden durch Oxidation mit Sauerstoff oder Sauerstoff enthaltenden Gasen.

Aldehyde sind die gebräuchlichen Ausgangsstoffe zur Gewinnung von Carbonsäuren. Die Vorzugsstellung für dieses Einsatzgebiet verdanken sie ihrer mannigfachen Verfügbarkeit und der leichten oxydativen Umwandlung der Carbonylgruppe in die Carboxylgruppe. Im Rahmen technisch ausgeübter Verfahren erfolgt die Umsetzung von Aldehyden zu Carbonsäuren vorwiegend in Anwesenheit von Katalysatoren. Es sind jedoch auch Prozesse bekannt, bei denen auf die Verwendung von Katalysatoren verzichtet wird. Zur Vermeidung von Nebenreaktionen arbeitet man sowohl bei den katalytischen als auch bei den nicht katalytischen Prozessen bei möglichst niedrigen Temperaturen, im allgemeinen wird eine Reaktionstemperatur von 100°C nicht überschritten. Als Katalysatoren kommen vorwiegend Salze von Übergangsmetallen in Betracht, insbesondere Salze des Kobalts und des Mangans sowie des Chroms, Eisens, Kupfers, Nickels, Silbers und Vanadiums. Häufig ist die Carbonsäurebildung aus Aldehyden, selbst bei Einhaltung optimaler Temperaturbedingungen, mit Neben- und Abbaureaktionen verbunden. Das gilt gleichermaßen für Reaktionen in Gegenwart wie in Abwesenheit von Katalysatoren. In solchen Fällen kann die Selektivität der Umsetzung durch Zusatz von Alkalimetallsalzen schwacher Säuren zu den Reaktanten erheblich verbessert werden. Nachteilig bei dieser Verfahrensvariante ist jedoch, dass die Salze inhibierend wirken, so dass für eine vollständige Umsetzung der Ausgangsstoffe die Reaktionszeit verlängert werden muss.

Nach dem in der DE-OS 30 29 700 beschriebenen Verfahren werden zur Herstellung von aliphatischen Monocarbonsäuren mit 6 bis 9 Kohlenstoffatomen die entsprechenden Aldehyde mit Sauerstoff in reiner Form oder mit Luft oxidiert. Als Katalysator wirkt eine Kombination von Mangan- und Kupferverbindungen, die in der Säure löslich sind, wobei das Molverhältnis von Mangan zu Kupfer im Bereich von 5:1 bis 0,5:1 liegt. Die Umsetzung der Ausgangsstoffe erfolgt in flüssiger Phase bei Temperaturen von etwa 50 bis 80°C und Drücken im Bereich von etwa 1,4 bis 10,3 bar. Als Hauptschwierigkeit dieses Verfahrens wird in der Prozessbeschreibung die Anwesenheit von Kupfer- und auch Manganverbindungen im Reaktionsprodukt, d.h. in der Carbonsäure, geschildert. Zur Entfernung der Metalle sind aufwendige Reinigungsmaßnahmen erforderlich, beispielsweise ihre Ausfällung mit wäßriger Oxalsäure.

Das in der US-Patentschrift 4 487 720 offenbarte Verfahren zur Herstellung von C₅- bis C₉-Monocarbonsäuren durch Oxidation von Aldehyden der gleichen Kohlenstoffatom-Zahl mit reinem Sauerstoff oder mit Luft arbeitet ebenfalls mit Kupfer- und Manganverbindungen als Katalysatoren. Als Nachteil dieser Arbeitsweise wird die Bildung von Kupferfilmen beschrieben, die bei der destillativen Reinigung der Säure auftreten und mechanische Schäden in der Destillationsapparatur zur Folge haben. Um dieses Problem zu vermeiden wird empfohlen, die Destillation in Gegenwart von Sauerstoff durchzuführen.

Ein weiteres katalytisches Verfahren zur Reaktion von Aldehyden mit Sauerstoff unter Bildung von Carbonsäuren ist Gegenstand der offengelegten internationalen Anmeldung WO97/14668. Als Katalysatoren finden substituierte oder unsubstituierte Alkylamine, Alkylamin-N-oxide, aromatische Amine, aromatische N-oxide, heterocyclische Amine, heterocyclische Amin-N-oxide und deren Mischungen Verwendung. Ausdrücklich wird darauf hingewiesen, dass die katalytisch wirkenden Stickstoffverbindungen einen im Vergleich zum Reaktionsprodukt höheren Siedepunkt besitzen müssen, um eine Verunreinigung der Säure durch den Katalysator zu unterbinden.

Nach der Lehre der offengelegten japanischen Patentanmeldung 53-105413 oxidiert man α-verzweigte aliphatische Aldehyde mit Sauerstoff in Gegenwart von Lithium- oder Erdalkalimetallverbindungen, die in Mengen von 0,01 bis 10 Gew.-% (bezogen auf das gesamte Reaktionssystem) eingesetzt werden, um α-verzweigte aliphatische Carbonsäuren herzustellen.

Kennzeichnend für die in der französischen Patentanmeldung 2 769 624 beschriebene Arbeitsweise ist die Einhaltung niedriger Reaktionstemperaturen, nämlich Temperaturen zwischen 0 und 25°C. Das Verfahren erfordert ebenfalls die Gegenwart von Alkalimetall- bzw. Erdalkalimetallverbindungen als Katalysatoren, die bei den einzuhaltenden, milden Temperaturen möglicherweise die Reaktionsgeschwindigkeit erhöhen. Denn lange Reaktionszeiten verbieten sich nicht nur aus wirtschaftlichen Gründen, sondern können auch zu unerwünschten Nebenreaktionen führen.

Nach der Lehre der DE 29 31 154 C2 stellt man aliphatische Carbonsäuren mit 4 bis 10 Kohlenstoffatomen durch Oxidation der entsprechenden Aldehyde mit Sauerstoff oder Sauerstoff enthaltenden Gasen in Gegenwart des komplexen Anions [Fe(CN)₅H₂O]³⁻ als Katalysator her. Wegen der thermischen Instabilität des Anions ist die Reaktionstemperatur auf einen Bereich von 20 bis 50°C beschränkt.

Gemische isomerer verzweigter aliphatischer oder cycloaliphatischer Carbonsäuren erhält man entsprechend der Arbeitsweise der deutschen Patentschrift 1 154 454 aus Aldehyden, die in dünner, flüssiger Schicht bei einer Temperatur von 65 bis 110°C ohne Verwendung von Katalysatoren mit einem sauerstoffhaltigen Gas oxidiert werden. Die Umsetzung wird in leeren oder mit inerten Stoffen großer Oberfläche gefüllten Rohren durchgeführt. Der Säureanteil im Reaktionsprodukt beträgt zwischen 59 und 80%.

Die bekannten Verfahren zur Herstellung von Carbonsäuren aus Aldehyden genügen noch nicht allen technischen und wirtschaftlichen Erfordernissen, die an moderne, industriell genutzte Prozesse gestellt werden. Die Anwendung von Katalysatoren ist mit aufwendigen Reinigungsschritten verbunden, denen das Reaktionsprodukt unterworfen werden muss, um Carbonsäuren zu erhalten, die problemlos weiterverarbeitet werden können. Nichtkatalytische Prozesse befriedigen häufig nicht hinsichtlich Umsatz und Selektivität zum gewünschten Produkt.

Es bestand daher die Aufgabe eine Arbeitsweise zu entwickeln, die die aufgezeigten Nachteile vermeidet und die Gewinnung von Carbonsäuren aus Aldehyden mit vertretbarem technischem Aufwand in hoher Ausbeute ermöglicht.

Diese Aufgabe wird gelöst durch ein Verfahren zur Herstellung aliphatischer Carbonsäuren mit 4 bis 10 Kohlenstoffatomen durch Oxidation der entsprechenden Aldehyde mit Sauerstoff oder Sauerstoff enthaltenden Gasen ohne Einsatz von Katalysatoren. Es ist dadurch gekennzeichnet, dass die Oxidation im Temperaturbereich von 0 bis 100°C in mindestens zwei Stufen, bei von Stufe zu Stufe ansteigenden Temperaturen, erfolgt.

Überraschenderweise gelingt es durch gezielte Temperaturführung, die Oxidation von Aldehyden zu Carbonsäuren mit Sauerstoff in reiner Form oder als Bestandteil von Gasgemischen, mit hohem Umsatz, sehr selektiv und ohne Einsatz von Katalysatoren durchzuführen. In diesem Zusammenhang muss insbesondere darauf hingewiesen werden, dass niedrige Reaktionstemperaturen, d.h. Temperaturen bis etwa 40°C, zu unbefriedigenden Umsätzen führen, höhere Reaktionstemperaturen, also Temperaturen oberhalb etwa 40°C, dagegen die Selektivität der Umsetzung deutlich beeinträchtigen. Unter diesen Umständen war nicht zu erwarten, dass die Kombination von Bereichen niedrigerer und höherer Reaktionstemperatur nicht zu einer Kumulation der unerwünschten Ergebnisse führt, sondern im Gegenteil sowohl Umsatz als auch Selektivität optimiert. Bemerkenswert ist ferner, dass durch Anwendung unterschiedlicher Temperaturen im Verlauf der Reaktion die Bildung von Perverbindungen, die unkontrollierte Oxidationsreaktionen auslösen können und dadurch die Selektivität der Umsetzung beeinträchtigen und überdies wegen ihrer leichten Zersetzbarkeit auch besondere Sicherheitsvorkehrungen erfordern, wirksam unterdrückt wird.

Ein sehr wesentliches Merkmal der erfindungsgemäßen Arbeitsweise ist die Wahl des Temperaturbereiches, in dem die Reaktion erfolgt und die Steuerung des Temperaturverlaufs während der Reaktion innerhalb dieses Intervalls. Es hat sich bewährt, die Aldehyde gemäß dem neuen Verfahren bei Temperaturen zwischen 0 und 100°C, vorzugsweise zwischen 20 und 100°C und insbesondere zwischen 40 und 80°C zu oxidieren. Im Gegensatz zur bisherigen Praxis wird die Umsetzung nicht bei einer konstanten oder, innerhalb der technischen Möglichkeiten, annähernd konstanten Temperatur vorgenommen, sondern in mehreren Stufen. Diese Stufen unterscheiden sich in der Weise voneinander, dass die Temperatur ansteigt, in der folgenden Stufe also höher ist als in der vorausgegangenen. Die Anzahl der Stufen ist grundsätzlich frei wählbar, sie kann daher den stofflichen Eigenarten der Einsatzaldehyde individuell angepasst werden, z.B. ihrem reaktiven Verhalten, ihrer Stabilität unter den Reaktionsbedingungen, aber auch ihrer Reinheit, sofern dieses Merkmal das Reaktionsgeschehen beeinflusst. Üblicherweise erfolgt die Aldehydoxidation erfindungsgemäß in zwei bis vier Stufen. Es können jedoch auch Verfahren mit mehr als vier Stufen praktiziert werden, obgleich sich solche Ausführungsformen im Hinblick auf den größeren apparativen Aufwand auf Sonderfälle beschränken. Im allgemeinen genügt es, das neue Verfahren als Zweistufenprozess auszugestalten, d.h. die Umsetzung der Reaktanten bei zwei Temperaturen vorzunehmen.

Die Temperaturdifferenz zwischen zwei aufeinander folgenden Stufen ist, abgesehen von der Art des Einsatzmaterials, im wesentlichen von zwei Faktoren abhängig, der Anfangstemperatur, also der Temperatur in der ersten Reaktionsstufe und der Gesamtzahl der Stufen. Je niedriger die Anfangstemperatur ist, desto größer kann, bei gegebener Stufenzahl, die Temperaturdifferenz sein. So hat es sich beispielsweise bei zweistufiger Arbeitsweise und einer Anfangstemperatur von 40°C bewährt, in der darauffolgenden Stufe eine Temperatur von 60 bis 70°C einzuhalten und bei einer Anfangstemperatur von 50°C die Reaktion in der zweiten Stufe bei 65 bis 75°C ablaufen zu lassen. Bei drei- und mehrstufigen Verfahren ist die Temperaturdifferenz zwischen benachbarten Stufen entsprechend geringer, sie kann zwischen 5 und 20°C, vorzugsweise 10 bis 15°C, betragen. Die genannten Temperaturangaben stellen lediglich Richtwerte dar, die im Einzelfall auf die speziellen Gegebenheiten abzustimmen sind. Werden mehr als zwei Reaktionsstufen angewandt, ist es nicht erforderlich, zwischen den einzelnen Stufen gleiche Temperaturunterschiede einzuhalten. Die Temperaturdifferenzen können vielmehr variabel gewählt und den individuellen Anforderungen angeglichen werden.

Die Verweilzeit der Aldehyde in den einzelnen Stufen des mehrstufigen Verfahrens ist abhängig von der gewählten Reaktionstemperatur. Allgemein gilt, dass man 40 bis 90 Gew.-% des eingesetzten Aldehyds bei Temperaturen bis 40°C und den Rest bei Temperaturen bis 100°C, vorzugsweise bis 80°C, umsetzt. Innerhalb der durch die vorstehend quantifizierten Maximaltemperaturen charakterisierten Bereiche können, wie oben beschrieben, weitere Reaktionsstufen vorgesehen werden. Dementsprechend können z.B. bei dreistufiger Reaktionsführung zwei Stufen bei Temperaturen bis 40°C und eine Stufe bis 100°C oder vorzugsweise 80°C aufeinander folgen. Ebenso ist es möglich, einen Teil des Aldehyds in einer bis maximal 40°C reichenden Temperaturstufe zur Carbonsäure umzusetzen und den restlichen Aldehyd in zwei Stufen bis 100°C, vorzugsweise 80°C, zu oxidieren. Entsprechend verfährt man bei vier- und mehrstufiger Arbeitsweise. Die Umsetzung wird bevorzugt bei Atmosphärendruck durchgeführt. Die Anwendung erhöhten Drucks ist jedoch nicht ausgeschlossen. Üblicherweise arbeitet man in einem Bereich von Atmosphärendruck bis 1,0 MPa, vorzugsweise bei Atmosphärendruck bis 0,8 MPa.

Die zur Umwandlung von Aldehyden in Carbonsäuren nach dem erfindungsgemäßen Verfahren erforderliche Reaktionszeit hängt unter anderem ab von der Reaktionstemperatur, der Art der Einsatzstoffe und dem Mengenverhältnis der Reaktanten. Normalerweise beträgt sie 30 min bis 20 h, insbesondere 3 bis 8 h.

Im Mittelpunkt des neuen Prozesses steht die Oxidation von unverzweigten und verzweigten C₄- bis C₁₀-Aldehyden, vorzugsweise von unverzweigten Aldehyden der genannten Molekülgröße. Die Herkunft der Aldehyde ist nicht auf bestimmte Herstellungsverfahren beschränkt. Aufgrund ihrer einfachen Zugänglichkeit werden durch Oxosynthese, d.h. durch Reaktion von C₃- bis C₉-Olefinen mit Kohlenmonoxid und Wasserstoff gewonnene Aldehyde bevorzugt. In diesem Zusammenhang ist es nicht entscheidend, welche spezielle Ausführungsform der Oxosynthese zur Gewinnung der Aldehyde diente, d.h. ob die Reaktion z.B. durch Kobalt oder durch Rhodium katalysiert wurde, ob man die Metalle allein oder zusammen mit Komplexbildnern einsetzte und der Katalysator im Reaktionsgemisch homogen gelöst war oder eine eigene, heterogene Phase bildete.

Als Oxidationsmittel verwendet man nach dem Verfahren der Erfindung molekularen Sauerstoff oder Gasgemische, die molekularen Sauerstoff enthalten. Weitere Bestandteile derartiger Gasgemische sind inerte Gase, z.B. Stickstoff, Edelgase und Kohlendioxid. Der Anteil der inerten Bestandteile des Sauerstoff enthaltenden Gasgemisches beträgt bis zu 90 Vol-%, insbesondere 30 bis 80 Vol-%. Die bevorzugten Oxidationsmittel sind Sauerstoff oder Luft.

Die Aldehyde können als solche oder gelöst in einem, unter den Reaktionsbedingungen inerten, Lösungsmittel eingesetzt werden. Beispiele für geeignete Lösungsmittel sind Ketone wie Aceton, Ester, z.B. Ethylacetat, Kohlenwasserstoffe, z.B. Toluol und Nitrokohlenwasserstoffe wie Nitrobenzol. Die Konzentration des Aldehyds wird durch seine Löslichkeit in dem Lösungsmittel begrenzt.

Das erfindungsgemäße Verfahren kann absatzweise oder kontinuierlich durchgeführt werden. Eine Rückführung nicht umgesetzter Reaktionsteilnehmer ist in beiden Fällen möglich. Bei absatzweisem Reaktionsablauf werden die Temperaturstufen durch Erhöhung der Temperatur im selben Reaktor nach Erreichen eines vorgegebenen Teilumsatzes (entsprechend einem vorgegebenen Temperatur-/Zeitprogramm) realisiert. Bei kontinuierlicher Prozessführung wird das Reaktionsgemisch ebenfalls nach Erreichen bestimmter Teilumsätze (oder gemäß einem vorbestimmten Temperatur-/Zeitprogramm) in zwei oder mehr als Kaskade angeordnete Reaktoren, die unterschiedliche Temperaturen aufweisen, eingeleitet.

Nach einer bewährten Ausführungsform des erfindungsgemäßen Verfahrens legt man den Aldehyd in einem geeigneten Reaktor, z.B. einem mit einem Anströmboden versehenen Rohrreaktor, der gegebenenfalls noch Füllkörper enthält, vor und leitet den Sauerstoff oder das Sauerstoff enthaltende Gasgemisch von unten durch den Aldehyd.

Entsprechend einer weiteren Ausführungsform verwendet man als Reaktor einen Rieselturm, der Füllkörper enthält. Über die Füllung läßt man den Aldehyd herabrieseln und leitet in den Turm gleichzeitig im Gleich- oder Gegenstrom Sauerstoff oder ein Sauerstoff enthaltendes Gasgemisch ein.

In den folgenden Beispielen wird die Herstellung von n-Pentansäure, n-Heptansäure und n-Nonansäure nach dem erfindungsgemäßen Verfahren beschrieben. Die Umsetzung läuft in zwei Stufen ab, die sich durch die jeweils eingehaltene Reaktionstemperatur unterscheiden. Den Beispielen werden die Ergebnisse von Vergleichsversuchen gegenübergestellt, die einstufig jeweils bei der tieferen bzw. bei der höheren Temperatur der zweistufigen Arbeitsweise durchgeführt wurden. Die Versuchsangaben enthalten
- die Auswaage an Oxidationsprodukt (Rohsäure); dieser Wert korreliert mit dem Umsatz;
- den Gehalt des Reaktionsproduktes an aktivem Sauerstoff; hierunter versteht man Sauerstoff in Form von Persäuren; aus Sicherheitsgründen soll der Persäuren-Anteil im Reaktionsprodukt möglichst gering sein;
- die GC-Analyse der Rohsäure; die Vorlauf- und die Nachlaufkomponenten sind nicht aufgegliedert, sondern unter den Begriffen Leichtsieder und Hochsieder zusammengefasst:

- den Aldehyd-Umsatz; bei isomerenhaltigen Einsatzgemischen werden ausschließlich die geradkettigen Aldehyde berücksichtigt;
- die Selektivität, d.h. den Carbonsäure-Anteil im Reaktionsprodukt, bezogen auf umgesetzten Aldehyd.

Selbstverständlich ist das neue Verfahren nicht auf die nachstehend beschriebenen Ausführungsformen beschränkt.

### Beispiele

### Herstellung von n-Pentansäure

### Vergleichsbeispiele 1 und 2

Die Flüssigphasenoxidationen von n-Pentanal zu n-Pentansäure wurden ohne Katalyatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40 µm.

In die Oxidationen wurden jeweils 800,0 g n-Pentanal folgender, durch gaschromatographische Analyse bestimmter, Zusammensetzung eingesetzt:

| | |
|---|---|
| 0,42 % | Leichtsieder |
| 99,07 % | n-Pentanal |
| 0,29 % | n-Pentansäure |
| 0,22 % | Hochsieder. |

Nach einer Oxidationszeit von 6 Stunden wurden folgende Ergebnisse ermittelt:

**Tabelle 1**

| Reaktionstemperatur (°C) | | Vergleichsbeispiel 1 40 | Vergleichsbeispiel 2 60 |
|---|---|---|---|
| Auswaage Oxidationsprodukt (g) | | 931,0 | 935,7 |
| Gehalt aktiver Sauerstoff (g/kg) | | 0,84 | 1,04 |
| GC-Analyse (%) | | | |
| | Leichtsieder | 0,31 | 0,46 |
| | n-Pentanal | 5,69 | 1,49 |
| | n-Pentansäure | 93,21 | 96,77 |
| | Hochsieder | 0,79 | 1,28 |
| | | | |
| Umsatz n-Pentanal (% d.Th.) | | 93,3 | 98,2 |
| Selektivität zu n-Pentansäure | | | |
| | (% d.Th.) | 99,3 | 98,7 |

### Beispiel 1

Dieser Versuch wurde unter den Bedingungen der Vergleichsbeispiele 1 und 2 durchgeführt mit der Abweichung, dass die Reaktionstemperatur im Verlauf der Oxidation verändert wurde. Zu Beginn der Reaktion wurde die Temperatur über 4 Stunden bei konstant 40°C gehalten, anschließend wurde über 2 Stunden ein Wert von 60°C eingestellt.

In die Oxidation wurden 800,0 g n-Pentanal der in den Vergleichsbeispielen 1 und 2 genannten Zusammensetzung eingesetzt.

Nach Abschluß der Oxidation (Reaktionszeit insgesamt 6 Stunden) wurden folgende Ergebnisse ermittelt:

| | | |
|---|---|---|
| Reaktionstemperatur (°C) | | |
| | 1. Stunde | 40 |
| | 2. Stunde | 40 |
| | 3. Stunde | 40 |
| | 4. Stunde | 40 |
| | 5. Stunde | 60 |
| | 6. Stunde | 60 |
| Auswaage Oxidationsprodukt (g) | | 936,9 |
| Gehalt aktiver Sauerstoff (g/kg) | | 0,50 |
| GC-Analyse (%); | | |
| | Leichtsieder | 0,29 |
| | n-Pentanal | 1,48 |
| | n-Pentansäure | 97,35 |
| | Hochsieder | 0,88 |
| | | |
| Umsatz n-Pentanal (% d.Th.) | | 98,2 |
| Selektivität zu n-Pentansäure (% d.Th.) | | 99,3 |

### Herstellung von n-Heptansäure

### Vergleichsbeispiele 3 und 4

Die Flüssigphasenoxidationen von n-Heptanal zu n-Heptansäure wurden ohne Katalysatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40 µm.

In die Oxidationen wurden jeweils 800,0 g n-Heptanal folgender, durch gaschromatographische Analyse bestimmter, Zusammensetzung eingesetzt:

| | |
|---|---|
| 0,62 % | Leichtsieder |
| 97,74 % | n-Heptanal |
| 0,10 % | n-Heptansäure |
| 1,54 % | Hochsieder. |

Nach einer Oxidationszeit von 6 Stunden wurden folgende Ergebnisse ermittelt:

**Tabelle 2**

| Reaktionstemperatur (°C) | | Vergleichsbeispiel 3 50 | Vergleichsbeispiel 4 70 |
|---|---|---|---|
| Auswaage Oxidationsprodukt (g) | | 899,2 | 902,9 |
| Gehalt aktiver Sauerstoff (g/kg) | | 0,71 | 1,54 |
| GC-Analyse (%) | | | |
| | Leichtsieder | 0,82 | 1,12 |
| | n-Heptanal | 5,42 | 2,15 |
| | n-Heptansäure | 91,79 | 94,05 |
| | Hochsieder | 1,97 | 2,68 |
| | | | |
| Umsatz n-Heptanal (% d.Th.) | | 93,8 | 97,5 |
| Selektivität zu n-Heptansäure | | | |
| | (% d.Th.) | 98,9 | 97,7 |

### Beispiel 2

Dieser Versuch wurde unter den Bedingungen der Vergleichsbeispiele 3 und 4 durchgeführt mit der Abweichung, dass die Reaktionstemperatur im Verlauf der Oxidation verändert wurde. Zu Beginn der Reaktion wurde die Temperatur über 4 Stunden bei konstant 50°C gehalten, anschließend wurde über 2 Stunden ein Wert von 70°C eingestellt.

In die Oxidation wurden 800,0 g n-Heptanal der in den Vergleichsbeispielen 3 und 4 genannten Zusammensetzung eingesetzt.

Nach Abschluß der Oxidation (Reaktionszeit insgesamt 6 Stunden) wurden folgende Ergebnisse ermittelt.

| | | |
|---|---|---|
| Reaktionstemperatur (°C) | | |
| | 1. Stunde | 50 |
| | 2. Stunde | 50 |
| | 3. Stunde | 50 |
| | 4. Stunde | 50 |
| | 5. Stunde | 70 |
| | 6. Stunde | 70 |
| Auswaage Oxidationsprodukt (g) | | 902,6 |
| Gehalt aktiver Sauerstoff (g/kg) | | 0,87 |
| GC-Analyse (%); | | |
| | Leichtsieder | 0,84 |
| | n-Heptanal | 2,20 |
| | n-Heptansäure | 94,85 |
| | Hochsieder | 2,11 |
| | | |
| Umsatz n-Heptanal (% d.Th.) | | 97,5 |
| Selektivität zu n-Heptansäure (% d.Th.) | | 98,8 |

### Herstellung von n-Nonansäure

### Vergleichsbeispiele 5 und 6

Die Flüssigphasenoxidationen von n-Nonanal zu n-Nonansäure wurden ohne Katalysatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40 µm.

In die Oxidationen wurden jeweils 800,0 g n-Nonanal folgender, durch gaschromatographische Analyse bestimmter, Zusammensetzung eingesetzt:

| | |
|---|---|
| 0,19 % | Leichtsieder |
| 9,61 % | Isononanal |
| 89,56 % | n-Nonanal |
| 0,04 % | Isononansäure |
| 0,25 % | n-Nonansäure |
| 0,35 % | Hochsieder |

Nach einer Oxidationszeit von 6 Stunden wurden folgende Ergebnisse ermittelt:

**Tabelle 3**

| Reaktionstemperatur (°C) | | Vergleichsbeispiel 5 50 | Vergleichsbeispiel 6 70 |
|---|---|---|---|
| Auswaage Oxidationsprodukt (g) | | 880,4 | 883,4 |
| Gehalt aktiver Sauerstoff (g/kg) | | 0,71 | 1,61 |
| GC-Analyse (%) | | | |
| | Leichtsieder | 0,40 | 1,10 |
| | Isononanal | 1,44 | 1,04 |
| | n-Nonanal | 7,27 | 2,88 |
| | Isononansäure | 8,15 | 7,90 |
| | n-Nonansäure | 81,78 | 85,44 |
| | Hochsieder | 0,96 | 1,64 |
| | | | |
| Umsatz n-Nonanal (% d.Th.) | | 91,1 | 96,4 |
| Selektivität zu n-Nonansäure | | | |
| | (% d.Th.) | 98,9 | 98,0 |

### Beispiel 3

Dieser Versuch wurde unter den Bedingungen der Vergleichsbeispiele 5 und 6 durchgeführt mit der Abweichung, dass die Reaktionstemperatur im Verlauf der Oxidation verändert wurde. Zu Beginn der Reaktion wurde die Temperatur über 4 Stunden bei konstant 50°C gehalten, anschließend wurde über 2 Stunden ein Wert von 70°C eingestellt.

In die Oxidation wurden 800,0 g n-Nonanal der in den Vergleichsbeispielen 5 und 6 genannten Zusammensetzung eingesetzt.

Nach Abschluß der Oxidation (Reaktionszeit insgesamt 6 Stunden) wurden folgende Ergebnisse ermittelt:

| | | |
|---|---|---|
| Reaktionstemperatur (°C) | | |
| | 1. Stunde | 50 |
| | 2. Stunde | 50 |
| | 3. Stunde | 50 |
| | 4. Stunde | 50 |
| | 5. Stunde | 70 |
| | 6. Stunde | 70 |
| Auswaage Oxidationsprodukt (g) | | 884,0 |
| Gehalt aktiver Sauerstoff (g/kg) | | 0,79 |
| GC-Analyse (%); | | |
| | Leichtsieder | 0,41 |
| | Isononanal | 1,00 |
| | n-Nonanal | 3,17 |
| | Isononansäure | 8,74 |
| | n-Nonansäure | 85,96 |
| | Hochsieder | 0,72 |
| | | |
| Umsatz n-Nonanal (% d.Th.) | | 96,1 |
| Selektivität zu n-Nonansäure (% d.Th.) | | 99,0 |

### Beispiel 4

Dieser Versuch wurde unter den Bedingungen der Vergleichsbeispiele 5 und 6 durchgeführt mit der Abweichung, dass die Reaktionstemperatur im Verlauf der Oxidation verändert wurde. Zu Beginn der Reaktion wurde die Temperatur über 5 Stunden bei konstant 50°C gehalten, anschließend wurde über 1 Stunde ein Wert von 70°C eingestellt.

In die Oxidation wurden 800,0 g n-Nonanal der in den Vergleichsbeispielen 5 und 6 genannten Zusammensetzung eingesetzt.

Nach Abschluß der Oxidation (Reaktionszeit insgesamt 6 Stunden) wurden folgende Ergebnisse ermittelt:

| | |
|---|---|
| Reaktionstemperatur (°C) | |
| 1. Stunde | 50 |
| 2. Stunde | 50 |
| 3. Stunde | 50 |
| 4. Stunde | 50 |
| 5. Stunde | 50 |
| 6. Stunde | 70 |
| Auswaage Oxidationsprodukt (g) | 884,8 |
| Gehalt aktiver Sauerstoff (g/kg) | 1,05 |
| GC-Analyse (%); | |
| Leichtsieder | 0,20 |
| Isononanal | 1,24 |
| n-Nonanal | 4,67 |
| Isononansäure | 8,67 |
| n-Nonansäure | 84,39 |
| Hochsieder | 0,83 |
| | |
| Umsatz n-Nonanal (% d.Th.) | 94,2 |
| Selektivität zu n-Nonansäure (% d.Th.) | 99,0 |

### Beispiel 5

Dieser Versuch wurde unter den Bedingungen der Vergleichsbeispiele 5 und 6 durchgeführt mit der Abweichung, dass die Reaktionstemperatur im Verlauf der Oxidation verändert wurde. Zu Beginn der Reaktion wurde die Temperatur über 3 Stunden bei konstant 50°C gehalten, anschließend wurde über 3 Stunden ein Wert von 70°C eingestellt.

In die Oxidation wurden 800,0 g n-Nonanal der in den Vergleichsbeispielen 5 und 6 genannten Zusammensetzung eingesetzt.

Nach Abschluß der Oxidation (Reaktionszeit insgesamt 6 Stunden) wurden folgende Ergebnisse ermittelt:

| | |
|---|---|
| Reaktionstemperatur (°C) | |
| 1. Stunde | 50 |
| 2. Stunde | 50 |
| 3. Stunde | 50 |
| 4. Stunde | 70 |
| 5. Stunde | 70 |
| 6. Stunde | 70 |
| Auswaage Oxidationsprodukt (g) | 886,8 |
| Gehalt aktiver Sauerstoff (g/kg) | 1,08 |
| GC-Analyse (%); | |
| Leichtsieder | 0,17 |
| Isononanal | 0,99 |
| n-Nonanal | 2,83 |
| Isononansäure | 8,75 |
| n-Nonansäure | 86,14 |
| Hochsieder | 1,12 |
| | |
| Umsatz n-Nonanal (% d.Th.) | 96,5 |
| Selektivität zu n-Nonansäure (% d.Th.) | 98,9 |

## Patentansprüche

1. Verfahren zur Herstellung aliphatischer Carbonsäuren mit 4 bis 10 Kohlenstoffatomen durch Oxidation der entsprechenden Aldehyde mit Sauerstoff oder Sauerstoff enthaltenden Gasen ohne Einsatz von Katalysatoren **dadurch gekennzeichnet, dass** die Oxidation im Temperaturbereich von 0 bis 100°C in mindestens zwei Stufen, bei von Stufe zu Stufe ansteigenden Temperaturen, erfolgt.

2. Verfahren nach Anspruch 1, **dadurch gekennzeichnet, dass** die Oxidation bei Temperaturen von 20 bis 100°C, insbesondere 40 bis 80°C, erfolgt.

3. Verfahren nach Anspruch 1 oder 2, **dadurch gekennzeichnet, dass** die Oxidation in 2 bis 4 Stufen, vorzugsweise in 2 Stufen, erfolgt.

4. Verfahren nach einem oder mehreren der Ansprüche 1 bis 3, **dadurch gekennzeichnet, dass** bei zweistufiger Arbeitsweise in der ersten Stufe eine Temperatur von 40°C und in der zweiten Stufe eine Temperatur von 60 bis 70°C eingehalten wird.

5. Verfahren nach einem oder mehreren der Ansprüche 1 bis 3, **dadurch gekennzeichnet, dass** bei zweistufiger Arbeitsweise in der ersten Stufe eine Temperatur von 50°C und in der zweiten Stufe eine Temperatur von 65 bis 75°C eingehalten wird.

6. Verfahren nach einem oder mehreren der Ansprüche 1 bis 5, **dadurch gekennzeichnet, dass** die Temperaturdifferenz zwischen benachbarten Temperaturstufen 5 bis 20°C, vorzugsweise 10 bis 15°C, beträgt.

7. Verfahren nach einem oder mehreren der Ansprüche 1 bis 6, **dadurch gekennzeichnet, dass** 40 bis 90 Gew.-% des eingesetzten Aldehyds bei Temperaturen bis 40°C und der Rest bei Temperaturen bis 100°C, vorzugsweise bis 80°C, umgesetzt werden.

8. Verfahren nach einem oder mehreren der Ansprüche 1 bis 7, **dadurch gekennzeichnet, dass** die Oxidation im Bereich von Atmosphärendruck bis 1,0 MPa, vorzugsweise im Bereich von Atmosphärendruck bis 0,8 MPa, erfolgt.

9. Verfahren nach einem oder mehreren der Ansprüche 1 bis 8, **dadurch gekennzeichnet, dass** das Oxidationsmittel molekularer Sauerstoff ist.

10. Verfahren nach einem oder mehreren der Ansprüche 1 bis 8, **dadurch gekennzeichnet, dass** das Oxidationsmittel ein molekularer Sauerstoff und bis zu 90 Vol-%, insbesondere 30 bis 80 Vol-% inerte Gase enthaltendes Gasgemisch ist.

11. Verfahren nach Anspruch 10, **dadurch gekennzeichnet, dass** das Gasgemisch Luft ist.

## Claims

1. A process for preparing aliphatic carboxylic acids having 4 to 10 carbon atoms by oxidation of the corresponding aldehydes with oxygen or oxygen-containing gases without the use of catalysts, wherein the oxidation takes place in the temperature range from 0 to 100°C in at least two stages, with the temperatures increasing from stage to stage.

2. The process as claimed in claim 1, wherein the oxidation takes place at temperatures of from 20 to 100°C, in particular 40 to 80°C.

3. The process as claimed in claim 1 or 2, wherein the oxidation takes place in two to four stages, preferably in two stages.

4. The process as claimed in one or more of claims 1 to 3, wherein in a two-stage procedure a temperature of 40°C is maintained in the first stage, and a temperature of 60 to 70°C is maintained in the second stage.

5. The process as claimed in one or more of claims 1 to 3, wherein in a two-stage procedure a temperature of 50°C is maintained in the first stage, and a temperature of 65 to 75°C is maintained in the second stage.

6. The process as claimed in one or more of claims 1 to 5, wherein the temperature difference between adjacent temperature stages is 5 to 20°C, preferably 10 to 15°C.

7. The process as claimed in one or more of claims 1 to 6, wherein 40 to 90% by weight of the aldehyde employed are reacted at temperatures up to 40°C, and the remainder is reacted at temperatures up to 100°C, preferably up to 80°C.

8. The process as claimed in one or more of claims 1 to 7, wherein the oxidation takes place in the range from atmospheric pressure to 1.0 MPa, preferably in the range from atmospheric pressure to 0.8 MPa.

9. The process as claimed in one or more of claims 1 to 8, wherein the oxidizing agent is molecular oxygen.

10. The process as claimed in one or more of claims 1 to 8, wherein the oxidizing agent is a gas mixture comprising molecular oxygen and up to 90% by volume, in particular 30 to 80% by volume, of inert gases.

11. The process as claimed in claim 10, wherein the gas mixture is air.

## Revendications

1. Procédé pour la préparation d'acides carboxyliques aliphatiques comprenant 4 à 10 atomes de carbone par oxydation des aldéhydes correspondants avec de l'oxygène ou des gaz contenant de l'oxygène sans utiliser de catalyseurs **caractérisé en ce que** l'oxydation est réalisée dans une plage de température de 0 à 100°C en au moins deux étapes, à une température qui augmente d'une étape à l'autre.

2. Procédé selon la revendication 1, **caractérisé en ce que** l'oxydation est réalisée à des températures de 20 à 100°C, en particulier de 40 à 80°C.

3. Procédé selon la revendication 1 ou 2, **caractérisé en ce que** l'oxydation est réalisée en 2 à 4 étapes, de préférence en 2 étapes.

4. Procédé selon l'une ou plusieurs des revendications 1 à 3, **caractérisé en ce qu'**on respecte, dans le cas d'un mode opératoire à deux étapes, dans la première étape, une température de 40°C et dans la deuxième étape une température de 60 à 70°C.

5. Procédé selon l'une ou plusieurs des revendications 1 à 3, **caractérisé en ce qu'**on respecte, dans le cas d'un mode opératoire à deux étapes, dans la première étape, une température de 50°C et dans la deuxième étape une température de 65 à 75°C.

6. Procédé selon l'une ou plusieurs des revendications 1 à 5, **caractérisé en ce que** la différence de température entre deux étapes de température adjacentes est de 5 à 20°C, de préférence de 10 à 15°C.

7. Procédé selon l'une ou plusieurs des revendications 1 à 6, **caractérisé en ce qu'**on transforme 40 à 90% en poids de l'aldéhyde utilisé à des températures jusqu'à 40°C et le reste à des températures jusqu'à 100°C, de préférence jusqu'à 80°C.

8. Procédé selon l'une ou plusieurs des revendications 1 à 7, **caractérisé en ce que** l'oxydation est réalisée dans la plage de la pression atmosphérique jusqu'à 1,0 MPa, de préférence dans la plage de la pression atmosphérique jusqu'à 0,8 MPa.

9. Procédé selon l'une ou plusieurs des revendications 1 à 8, **caractérisé en ce que** l'oxydant est l'oxygène moléculaire.

10. Procédé selon l'une ou plusieurs des revendications 1 à 8, **caractérisé en ce que** l'oxydant est un mélange gazeux contenant de l'oxygène moléculaire et jusqu'à 90% en volume, en particulier 30 à 80% en volume de gaz inertes.

11. Procédé selon la revendication 10, **caractérisé en ce que** le mélange gazeux est l'air.
